# EUROPEAN PATENT SPECIFICATION

(11) **EP 2 027 862 B1**
(45) Date of publication and mention of the grant of the patent: **08.04.2015**
(21) Application number: 06766679.2
(22) Date of filing: 14.06.2006
(51) Int. Cl.: A61K 31/702, A23L 1/30, A23L 1/304, A61K 8/27, A61K 8/60, A61K 31/7016, A61K 33/30, A61P 37/04, A61P 43/00, A23K 1/16, A61K 45/06, A61Q 19/00, A23K 1/175

(54) **COMPOSITION FOR ENHANCING IMMUNE FUNCTION COMPRISING ZINC AND A NIGEROOLIGOSACCHARIDE**
ZUSAMMENSETZUNG ZUR VERBESSERUNG DER IMMUNFUNKTION MIT ZINK UND EINEM NIGEROOLIGOSACCHARID
COMPOSITION POUR AUGMENTER LA FONCTION IMMUNITAIRE COMPRENANT DU ZINC ET UN NIGEROOLIGOSACCHARIDE

(43) Date of publication of application: 25.02.2009
(73) Proprietor: House Wellness Foods Corporation, Hyogo 664-0011 (JP)
(72) Inventor: KOYAMA, Yohei, Kawanishi-shi, Hyogo 666-0023 (JP); MUROSAKI, Shinji, Nara-shi, Nara 630-8114 (JP); YAMAMOTO, Yoshihiro, Itami-shi, Hyogo 664-0002 (JP); YAMAMOTO, Norio, Kobe-shi, Hyogo 658-0066 (JP); HIROSE, Yoshitaka, Itami-shi, Hyogo 664-0001 (JP)
(74) Representative: Teipel, Stephan
(86) International application number: PCT/JP2006/311910
(87) International publication number: WO 2007/144943

(56) References cited:
- EP-A1- 1 520 584
- WO-A2-02/069985
- JP-A- 9 227 382
- JP-A- 09 052 834
- JP-A- 2002 080 364
- JP-A- 2002 512 966
- US-A- 6 139 864
- SHINJI MUROSAKI ET AL: "Nigerooligosaccharides augments natural killer activity of hepatic mononuclear cells in mice", INTERNATIONAL IMMUNOPHARMACOLOGY, vol. 2, no. 1, 1 January 2002 (2002-01-01) , pages 151-159, XP55041924, ISSN: 1567-5769, DOI: 10.1016/S1567-5769(01)00152-7
- HIROSE YOSHITAKA ET AL: "Nigerooligosaccharides augments mitogen-induced proliferation and suppresses activation-induced apoptosis of human peripheral blood mononuclear cells", IMMUNOPHARMACOLOGY AND IMMUNOTOXICOLOGY, vol. 26, no. 3, 2004, pages 387-399, XP9164013, ISSN: 0892-3973
- MUROSAKI S. ET AL.: 'Immunoptentiating activity of nigerooligosaccharides for the T helper 1-like immune response in mice' BIOSCI. BIOTECHNOL. BIOCHEM. vol. 63, no. 2, 1999, pages 373 - 378, XP003020863
- ARAKAWA Y.: 'Biryo Genso kara mita Seitai Kino to sono Ijo' BIOMED. RES. TRACE ELEMENTS vol. 6, no. 3, 1995, pages 135 - 136, XP003020864
- FLYNN A.: 'In vitro levels of copper, magnesium and zinc required for mitogen stimulated T lymphocyte proliferation' NUTR. RES. vol. 5, 1985, pages 487 - 495, XP003020865

## Description

### TECHNICAL FIELD

The present invention relates to a composition for enhancing immune function comprising, as active ingredients, zinc and a saccharide having 3-O-α-D-glucopyranosyl-D-glucose as a structural unit.

### BACKGROUND ART

Saccharides having 3-O-α-D-glucopyranosyl-D-glucose as a structural unit have been reported to exhibit immunostimulatory effect (see also Biosci. Biotechnol. Biochem. , 1999, 63:373-378 and Int. Immunopharmacol., 2002, 2:151-159). Compositions comprising such a saccharide as an active ingredient are disclosed as an immunostimulator (see also JP-A Nos. 9-52834, 2001-64174 and 2002-80364), a natural killer cell activator (see also JP-A No. 2002-265366), a QOL enhancer (see also JP-A No. 2002-265385), etc.

Meanwhile, zinc is the second most abundant element, following iron among trace elements present in human body. Various reports have been made on the physiological function of zinc (see also J. Leukoc. Biol., 2002, 71:25-27), and zinc plays an important role on protein synthesis as an enzyme component, sense of taste and smell, insulin production and function, and reproductive function and immunostimulation. In particular, zinc is known to play wide-ranging roles in the general immune function, for example, to regulate the functions of lymphocytes such as the activation of T lymphocytes or natural killer cells (see also J. Nutr. 2003, 133:1452S-1456S).

### DISCLOSURE OF THE INVENTION

### PROBLEM TO BE SOLVED BY THE INVENTION

The object of the present invention is to provide a composition having a more excellent immunoenhancing effect.

### MEANS FOR SOLVING THE PROBLEM

After intensive investigations, the present inventors found that the combined administration of zinc and a saccharide having S3-O-α-D-glucopyranosyl-D-glucose as a structural unit exhibited a remarkably superior immunoenhancing effect, compared to the independent administration of each ingredient. They have carried out further investigations and completed the present invention.

Namely, the present invention relates to the following.
(1) A composition for enhancing immune function comprising, as active ingredients, zinc and a saccharide having 3-O-α-D-glucopyranosyl-D-glucose as a structural unit,
   wherein the saccharide is nigerooligosaccharide comprising at least one of nigerose, nigerosyl glucose and nigerosyl maltose.
(2) The composition for enhancing immune function according to the above (1), wherein a weight ratio of the zinc to the saccharide is 1:200 to 1:2000.
(3) The composition for enhancing immune function according to any one of the above (1) or (2), wherein enhancing immune function is enhancing metabolic activity of an immunocompetent cell.
(4) The composition for enhancing immune function according to any one of the above (1) or (2), wherein enhancing immune function is enhancing growth potential of an immunocompetent cell.
(5) The composition for enhancing immune function according to any one of the above (1) or (2), wherein enhancing immune function is enhancing metabolic activity of a T lymphocyte.
(6) The composition for enhancing immune function according to any one of the above (1) or (2), wherein enhancing immune function is enhancing growth potential of a T lymphocyte.
(7) The composition for enhancing immune function according to any one of the above (1) or (2), wherein enhancing immune function is inhibiting cell death of a T lymphocyte.
(8) The composition for enhancing immune function according to any one of the above (1) to (7), which is a pharmaceutical product or a food product.
(9) food product comprising the composition for enhancing immune function according to any one of the above (1) to (7).
(10) A feed product comprising the composition for enhancing immune function according to any one of the above (1) to (7).
(11) A cosmetic product comprising the composition for enhancing immune function according to any one of the above (1) to (7).

### EFFECT OF THE INVENTION

The composition for enhancing immune function according to the present invention exhibits a remarkably superior immunoenhancing effect to that achieved by the independent use of zinc and a saccharide having 3-O-α-D-glucopyranosyl-D-glucose as a structural unit. The composition therefore can maintain and promote health more effectively.

### BEST MODE FOR CARRYING OUT THE INVENTION

The composition for enhancing immune function according to the present invention comprises, as active ingredients, zinc and a saccharide having 3-O-α-D-glucopyranosyl-D-glucose as a structural unit.

A specific example of the saccharide is an oligosaccharide comprising at least one or more α-1,3 glucosidic bonds and having a degree of glucose polymerization of about 2 or more. Preferred is nigerooligosaccharide having a degree of glucose polymerization of about 2 to 10, and more preferred is nigerooligosaccharide having a degree of glucose polymerization of about 2 to 7. The nigerooligosaccharide also includes an oligosaccharide comprising an α-1,3 glucosidic bond and other bonds (for example, α-1,1, α-1,2, α-1,4, α-1,6 glucosidic bonds, etc.) in addition to an oligosaccharide comprising an α-1,3 glucosidic bond only. In the present invention, it is required to use nigerose, nigerosyl glucose or nigerosyl maltose represented by each of the following formulae. These may be used alone or in combination of two or more.

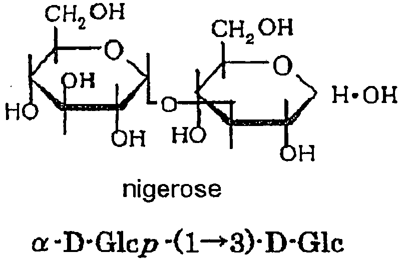

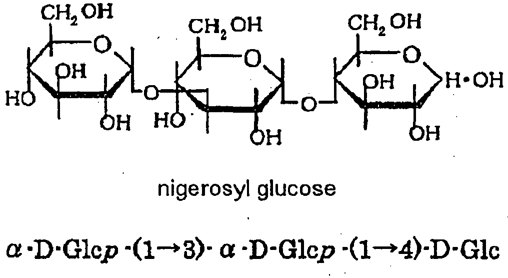

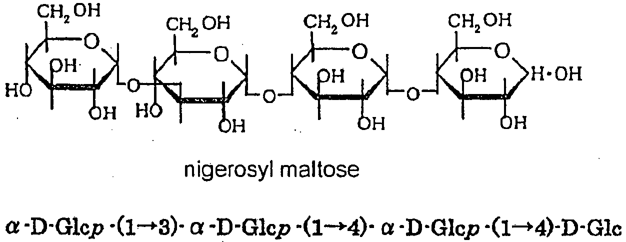

The saccharide having 3-O-α-D-glucopyranosyl-D-glucose as a structural unit can be easily prepared in accordance with a method known per se. Specifically, nigerooligosaccharide can be prepared by the following known methods, for example. For example, a method of preparing nigerooligosaccharide by hydrolyzing, using an enzyme, an acid, etc., a substrate such as nigeran or elsinan, etc. , which is a polysaccharide produced in microorganism, is described in M. Stacey and J.M. Webber: Methods in Carbohydrate Chemistry, I, 339-341, AcademicPress (1962). Moreover, a method of preparing nigerose through known α-glucosidase-catalyzed transglycosylation and condensation is also known (see also Ken-ichi KANAYA, et al., Japan Society for Bioscience, Biotechnology and Agrochemistry, 53, 385-390 (1979) and H. FUJIMOTO, et al., Agric. Biol. Chem., 52, 1345-1351 (1988), etc.). Further, a method of preparing nigerose by reacting starch hydrolysate with cyclodextrin glucanotransferase is disclosed by JP-A No. 3-22958. Furthermore, JP-A No. 7-59559 discloses a method of preparing nigerooligosaccharide by reacting a substrate containing polysaccharide or oligosaccharide each comprising an α-1,4 glucosidic bond with a glycosyltransferase which forms an α-1,3 glucosidic bond (specifically, the glycosyltransferase is prepared by cultivating the fungus of the genus Acremonium producing a glycosyltransf erase which forms an α-1,3 glucosidic bond, for example, Acremonium sp. S4G13 (FERM BP-4373) in accordance with a conventional method). The nigerooligosaccharide used in the present invention can be prepared by any of the above-mentioned methods, but it is not limited thereto. However, the most economical in the known methods so far is probably the method disclosed in the above-mentioned JP-A No. 7-59559, comprising the use of the glycosyltransferase (nigerooligosaccharide forming enzyme). The nigerooligosaccharide prepared in accordance with this method is preferably used in the present invention as well.

Meanwhile, the zinc, which is an active ingredient of the composition for enhancing immune function according to the present invention, is usually provided as a zinc salt (for example, zinc sulfate, zinc nitrate, zinc phosphate, etc.).

The proportion of zinc to saccharide having 3-O-α-D-glucopyranosyl-D-glucose as a structural unit (hereinafter also referred to as just saccharide), both of which are active ingredients, is not limited in particular and, for example, the weight ratio of zinc relative to saccharide (for example, nigerooligosaccharide etc.) is about 1:100 to 1:5000, preferably about 1:200 to 1:2000, more preferably about 1:500 to 1:1500. The composition for enhancing immune function according to the present invention can be prepared by mixing saccharide with zinc by a method known per se (for example, mixing with stirring etc.).

The administration of the composition for enhancing immune function according to the present invention to a mammal and a fish can enhance the immune function thereof. The mammal is preferably a human, although it is not limited thereto. It may be livestock such as cattle, a horse, a pig, a sheep and a goat; poultry such as a chicken; or a pet animal such as a dog and a cat. The fish is preferably a farmed fish, such as a yellowtail.

Upon use of the composition for enhancing immune function according to the present invention as a pharmaceutical product, it can be administered orally or parenterally. For example, the daily dose in terms of the amount of saccharide, an active ingredient, may be about 4 mg to 40 g, preferably about 10 mg to 20 g, more preferably about 50 mg to 10 g for an adult weighing about 60 kg, although it depends on subjects to be administered, administration routes and patient conditions, etc. A non-human mammal or a fish also can be administered with the same dose per unit of body weight as above.

Upon use of the composition for enhancing immune function according to the present invention as a pharmaceutical product, the dosage form may be oral preparations (powders, granules, pills, tablets, hard capsules, soft capsules, syrups, etc.) or parenteral preparations such as suppositories or injections. These preparations can be prepared by a method known per se.

Moreover, the composition for enhancing immune function according to the present invention can be used per se as a food product (especially a functional food product) or can be incorporated into a food or drink product, or a feed product. The food or drink product includes, for example, sweeteners, confectionery, gum, candy, jelly, tea or juice. The feed product includes, for example, feed for a non-human mammal or a fish.

With regard to the amount of the composition for enhancing immune function according to the present invention in the food or drink product, or the feed product, the amount of saccharide, an active ingredient, is preferably about 1 to 80% (W/W) relative to the total amount of the food or drink product, or the feed product, for example.

Moreover, the composition for enhancing immune function according to the present invention can also be used as a cosmetic product containing the same. The cosmetic product includes, for example, lotion for external use, milky lotion for external use, cream for external use or gel for external use.

With regard to the amount of the composition for enhancing immune function according to the present invention in the cosmetic product, the amount of saccharide, an active ingredient, is preferably about 1 to 20% (W/W) relative to the total amount of the cosmetic product, for example.

The immunoenhancing effect exhibited by the composition for enhancing immune function according to the present invention includes the effect of enhancing the metabolic activity or growth potential of immunocompetent cells, or the effect of enhancing the metabolic activity or growth potential of T lymphocytes or inhibiting cell death of T lymphocytes, for example.

The composition for enhancing immune function according to the present invention is effective for preventing and treating infectious diseases caused by microorganisms such as viruses and bacteria, including, for example, infectious enteritis via oral infection with Vibrio cholera, enterotoxigenic Escherichia coli, Shigella, Salmonella, viruses, etc.; influenza and cold syndrome via respiratory tract infection; and stomatitis and periodontal diseases via intraoral infection, as well as malignant tumors, including, for example, epithelial malignant tumors generated in gastrointestinal tract, respiratory mucous membrane and parenchymal organs such as liver and kidney; and nonepithelial malignant tumors generated in locomotive organs and soft tissues.

### EXAMPLE

Hereinafter, the present invention will be illustrated in detail by Test Examples and Examples, but it is not limited thereto.

### Test Example 1

The effect of the combined use of zinc and nigerooligosaccharide on the metabolic activity of immunocompetent cells was assessed using zinc and nigerooligosaccharide, which is a saccharide having 3-O-α-D-glucopyranosyl-D-glucose as a structural unit. Zinc was used in the form of zinc sulfate dissolved in distilled water. Spleens were aseptically harvested from ten-week-old female Balb/c mice, teased in RPMI1640 culture medium (GIBCO) supplemented with streptomycin and penicillin (SIGMA), and then passed through a #200 mesh screen to obtain spleen cell suspension of immunocompetent cells. After counting the cells in the spleen cell suspension with an automatic blood cell counter (Sysmex Corporation: type CDA-500), the suspension was adjusted to a concentration of 1 x 10⁷ cells/ml in the above-mentioned RPMI1640 culture medium and plated in a volume of 50 µl per well into a 96-well culture plate. Concanavalin A (SIGMA), a cell-growth stimulating factor, was dissolved in the above-mentioned RPMI1640 culture medium, and 50 µl of the Concanavalin A solution was added to the cell suspension in each well to give a final concentration of 2.0 µg/ml. Additionally, zinc and nigerooligosaccharide were separately dissolved in the above-mentioned RPMI1640 culture medium. 50 µl of the zinc solution was added to each well to give a final concentration of 0 or 1 ng/ml, and similarly, 50 µl of the nigerooligosaccharide solution was added to each well to give a final concentration of 0 or 1000 ng/ml. Thus-prepared immunocompetent cells were cultured in an incubator with 5% CO₂ at 37°C for 48 hours. After the incubation, WST-1 (2-(4-indophenyl)-3-(4-nitrophenyl)-5-(2,4-disulfophenyl)-2 H-tetrazolium, monosodium salt; Dojindo Laboratories) was dissolved at a concentration of 10 mM in 0.4 mM 1-methoxy PMS (Dojindo Laboratories), and 10 µl of the WST-1 solution was added to each well. During 3-hour incubation in the CO₂ incubator, water-soluble formazan was produced from WST-1, a tetrazolium salt, via mitochondrial dehydrogenase of a living cell. The concentration of the produced formazan was used as the index of the metabolic activity of immunocompetent cells. The formazan concentration was determined from absorbance at a measurement wavelength of 415 nm (reference wavelength of 630 nm) using a microplate reader (Corona Electric Co., Ltd. : Type MTP-32). The results are shown in Table 1.

**Table 1**

| | | zinc(ng/ml) | |
|---|---|---|---|
| | | 0 | 1 |
| Nigerooligosaccharide (ng/ml) | 0 | 0.800±0.030 | 0.815±0.013 |
| | 1000 | 0.811±0.008 | 0.857±0.019* |

| | | | |
|---|---|---|---|
| The values in Table 1 represent absorbance at 415 nm as means ± SD of triplicate wells. The symbol "*" indicates significant difference from the group not treated with either zinc or nigerooligosaccharide (Dunnett's test). | | | |

As Table 1 clearly shows, the combined use of nigerooligosaccharide and zinc significantly raised the metabolic activity of mouse immunocompetent cells under the condition where the independent use of nigerooligosaccharide and zinc, each of which has immunoenhancing effect, hardly raised the metabolic activity. Such a rise in cell metabolic activity is known to greatly reflect cell growth, and cell growth is crucial for the functional expression of immunocompetent cells, in particular, lymphocytes such as T lymphocytes and B lymphocytes. This revealed that the combined use of nigerooligosaccharide and zinc had an evident immunoenhancing effect. Furthermore, since the rise in cell metabolic activity also reflects cell death inhibition, the present composition was shown to have immunoenhancing effect through such a mechanism as well.

### Test Example 2

In this Test Example, the effect of the combined use of nigerooligosaccharide and zinc on the metabolic activity of immunocompetent cells during prolonged culture was assessed using nigerooligosaccharide and zinc in the same manner as Test Example 1. Spleens were aseptically harvested from ten-week-old female Balb/c mice, teased in RPMI1640 culture medium (GIBCO) supplemented with streptomycin and penicillin (SIGMA), and then passed through a #200 mesh screen to obtain spleen cell suspension of immunocompetent cells. After counting the cells in the spleen cell suspension with an automatic blood cell counter (Sysmex Corporation: type CDA-500), the suspension was adjusted to a concentration of 1 x 10⁷ cells/ml in the above-mentioned RPMI1640 culture medium and plated in a volume of 50 µl per well into a 96-well culture plate. Concanavalin A (SIGMA), a cell-growth stimulating factor, was dissolved in the above-mentioned RPMI1640 culture medium, and 50 µl of the Concanavalin A solution was added to the cell suspension in each well to give a final concentration of 0.4 µg/ml. Additionally, zinc and nigerooligosaccharide were separately dissolved in the above-mentioned RPMI1640 culture medium. 50 µl of the zinc solution was added to each well to give a final concentration of 0, 1 or 2 ng/ml, and similarly, 50 µl of the nigerooligosaccharide solution was added to each well to give a final concentration of 0 or 1000 ng/ml. Thus-prepared immunocompetent cells were cultured in an incubator with 5% CO₂ at 37°C for 144 hours. After the incubation, WST-1 (2-(4-indophenyl)-3-(4-nitrophenyl)-5-(2,4-disulfophenyl)-2 H-tetrazolium, monosodium salt; Dojindo Laboratories) was dissolved at a concentration of 10 mM in 0.4 mM 1-methoxy PMS

(Dojindo Laboratories), and 10 µl of the WST-1 solution was added to each well. During 4.5-hour incubation in the CO₂ incubator, water-soluble formazan was produced from WST-1, a tetrazolium salt, via mitochondrial dehydrogenase of a living cell. The concentration of the produced formazan was used as the index of the metabolic activity of immunocompetent cells. The formazan concentration was determined from absorbance at a measurement wavelength of 415 nm (reference wavelength of 630 nm) using a microplate reader (Corona Electric Co., Ltd. : Type MTP-32). The results are shown in Table 2.

**Table 2**

| | | zinc(ng/ml) | | |
|---|---|---|---|---|
| | | 0 | 1 | 2 |
| Nigerooligosaccharide (ng/ml) | 0 | 0.772 | 0.793 | 0.786 |
| | 1000 | 0.791 | 0.813 | 0.827 |

| | | | | |
|---|---|---|---|---|
| The values in Table 2 represent absorbance at 415 nm as means of triplicate to hexplicate wells. | | | | |

As Table 2 clearly shows, the combined use of nigerooligosaccharide and zinc in the proportion of 1:1000 or 1:500 during prolonged culture raised the metabolic activity of mouse immunocompetent cells in a zinc dose-dependent manner, under the condition where the independent use of nigerooligosaccharide and zinc hardly raised the metabolic activity. Namely, the present composition was shown to have immunoenhancing effect on mouse immunocompetent cells during prolonged culture as well.

### Test Example 3

In the same manner as in Test Example 1, the synergic effect of nigerooligosaccharide and zinc on the metabolic activity of human peripheral mononuclear cells was assessed using nigerooligosaccharide and zinc. After 20 ml of blood collected from each healthy male adult was diluted 2-fold with calcium-and magnesium-free phosphate buffered saline (PBS), the diluted blood sample was overlaid onto 30 ml of a Ficoll-Paque PLUS (GE Healthcare Bio-Sciences) layer so gently as not to disturb the interface, followed by centrifugation at 400 x g at 20°C for 40 minutes. After the centrifugation, lymphocytes and monocytes present in the intermediate layer were carefully collected in a Pasteur pipette. The collected cell suspension was washed with 10-fold PBS twice. After the obtained mononuclear cells were counted with an automatic blood cell counter (Sysmex Corporation: type CDA-500), the suspension was adjusted to a concentration of 2 x 10⁶ cells/ml in RPMI1640 culture medium supplemented with 10% fetal bovine serum, streptomycin and penicillin, and plated in a volume of 50 µl per well into a 96-well culture plate. Concanavalin A (SIGMA) was dissolved in the above-mentioned RPMI1640 culture medium, and 50 µl of the Concanavalin A solution was added to the cell suspension in each well to give a final concentration of 2.0 µg/ml. Additionally, zinc and nigerooligosaccharide were separately dissolved in the above-mentioned RPMI1640 culture medium. 50 µl of the zinc solution was added to each well to give a final concentration of 0 or 10 ng/ml, and similarly, 50 µl of the nigerooligosaccharide solution was added to each well to give a final concentration of 0 or 10 µg/ml. Thus-prepared human peripheral mononuclear cells were cultured in an incubator with 5% CO₂ at 37°C for 48 hours. After the incubation, WST-1 (Dojindo Laboratories) was dissolved at a concentration of 10 mM in 0.4 mM 1-methoxy PMS (Dojindo Laboratories), and 10 µl of the WST-1 solution was added to each well. During 1.5-hour incubation in the CO₂ incubator, water-soluble formazan was produced from WST-1, a tetrazolium salt, via mitochondrial dehydrogenase of a living cell. The concentration of the produced formazan was used as the index of the metabolic activity of human peripheral mononuclear cells. The formazan concentration was determined from absorbance at a measurement wavelength of 415 nm (reference wavelength of 630 nm) using a microplate reader (Corona Electric Co., Ltd. : Type MTP-32). The results are shown in Table 3.

**Table 3**

| | | zinc(ng/ml) | |
|---|---|---|---|
| | | 0 | 10 |
| Nigerooligosaccharide (µg/ml) | 0 | 0.350±0.005 | 0.348±0.003 |
| | 10 | 0.358±0.007 | 0.367±0.012* |

| | | | |
|---|---|---|---|
| The values in Table 3 represent absorbance at 415 nm as means ± SD of triplicate wells. The symbol "*" indicates significant difference from the group not treated with either zinc or nigerooligosaccharide (Dunnett's test). | | | |

As Table 3 clearly shows, the combined use of nigerooligosaccharide and zinc in the proportion of 1:1000 significantly raised the metabolic activity of human immunocompetent cells, under the condition where the independent use of nigerooligosaccharide and zinc hardly raised the metabolic activity. Namely, this test employing human peripheral mononuclear cells also revealed that a composition comprising nigerooligosaccharide and zinc had immunoenhancing effect.

### Example 1

All the ingredients were well mixed in the amounts described in the following Table 4 and the mixture was compressed into tablets so that each tablet weighs 700 mg.

**Table 4**

| Ingredient | Amount (% by weight) |
|---|---|
| Nigerooligosaccharide | 14 |
| Zinc sulfate(in terms of zinc) | 0.07 |
| Lactose | 81 |
| Crystalline cellulose | 1 |
| Talc | 4 |

### Example 2

All the ingredients were well mixed in the amounts described in the following Table 5 to give powdered concentrate feed for dairy cows.

**Table 5**

| Ingredient | Amount (% by weight) |
|---|---|
| Nigerooligosaccharide | 10 |
| Zinc sulfate(in terms of zinc) | 0.005 |
| Rolled barley | 20 |
| Wheat bran | 23 |
| Rolled corn | 15 |
| Soymeal | 9 |
| Soy hull | 9 |
| Rolled soy | 7 |
| Cotton seed | 7 |

### Example 3

All the ingredients were well mixed in the amounts described in the following Table 6 and then soft candies were prepared so that each candy weighs 5 g.

**Table 6**

| Ingredient | Amount (% by weight) |
|---|---|
| Nigerooligosaccharide | 8 |
| Zinc sulfate(in terms of zinc) | 0.008 |
| Granulated sugar | 9 |
| Starch syrup | 20 |
| Milk | 50 |
| Fresh cream | 9 |
| Fondant cream | 3.5 |
| Sugar ester | 0.5 |

### INDUSTRIAL APPLICABILITY

The composition for enhancing immune function according to the present invention exhibits a remarkably superior immunoenhancing effect to that achieved by the independent use of zinc and a saccharide having 3-O-α-D-glucopyranosyl-D-glucose as a structural unit.

## Claims

1. A composition for enhancing immune function comprising, as active ingredients, zinc and a saccharide having 3-O-α-D-glucopyranosyl-D-glucose as a structural unit, wherein the saccharide is nigerooligosaccharide comprising at least one of nigerose, nigerosyl glucose and nigerosyl maltose.

2. The composition for enhancing immune function according to Claim 1, wherein a weight ratio of the zinc to the saccharide is 1:200 to 1:2000.

3. The composition for enhancing immune function according to Claim 1 or 2, wherein enhancing immune function is enhancing metabolic activity of an immunocompetent cell.

4. The composition for enhancing immune function according to Claim 1 or 2, wherein enhancing immune function is enhancing growth potential of an immunocompetent cell.

5. The composition for enhancing immune function according to Claim 1 or 2, wherein enhancing immune function is enhancing metabolic activity of a T lymphocyte.

6. The composition for enhancing immune function according to Claim 1 or 2, wherein enhancing immune function is enhancing growth potential of a T lymphocyte.

7. The composition for enhancing immune function according to Claim 1 or 2, wherein enhancing immune function is inhibiting cell death of a T lymphocyte.

8. The composition for enhancing immune function according to any one of Claims 1 to 7, which is a pharmaceutical product or a food product.

9. A food product comprising the composition for enhancing immune function according to any one of Claims 1 to 7.

10. A feed product comprising the composition for enhancing immune function according to any one of Claims 1 to 7.

11. A cosmetic product comprising the composition for enhancing immune function according to any one of Claims 1 to 7.

## Patentansprüche

1. Zusammensetzung zur Verbesserung der Immunfunktion, umfassend als Wirkstoffe Zink und ein Saccharid mit einer 3-O-α-D-Glucopyranosyl-D-glucose als eine strukturelle Einheit, wobei das Saccharid Nigerooligosaccharid, umfassend mindestens eines von Nigerose, Nigerosylglucose und Nigerosylmaltose, ist.

2. Zusammensetzung zur Verbesserung der Immunfunktion nach Anspruch 1, wobei das Gewichtsverhältnis des Zinks zu dem Saccharid 1 : 200 bis 1 : 2000 beträgt.

3. Zusammensetzung zur Verbesserung der Immunfunktion nach Anspruch 1 oder 2, wobei die Verbesserung der Immunfunktion das Verbessern der Stoffwechselaktivität einer immunkompetenten Zelle ist.

4. Zusammensetzung zur Verbesserung der Immunfunktion nach Anspruch 1 oder 2, wobei die Verbesserung der Immunfunktion das Verbessern des Wachstumspotentials einer immunkompetenten Zelle ist.

5. Zusammensetzung zur Verbesserung der Immunfunktion nach Anspruch 1 oder 2, wobei die Verbesserung der Immunfunktion das Verbessern der Stoffwechselaktivität eines T-Lymphozyten ist.

6. Zusammensetzung zur Verbesserung der Immunfunktion nach Anspruch 1 oder 2, wobei die Verbesserung der Immunfunktion das Verbessern des Wachstumspotentials eines T-Lymphozyten ist.

7. Zusammensetzung zur Verbesserung der Immunfunktion nach Anspruch 1 oder 2, wobei die Verbesserung der Immunfunktion das Inhibieren des Zelltods eines T-Lymphozyten ist.

8. Zusammensetzung zur Verbesserung der Immunfunktion nach einem der Ansprüche 1 bis 7, welche ein pharmazeutisches Produkt oder ein Nahrungsmittelprodukt ist.

9. Nahrungsmittelprodukt, umfassend die Zusammensetzung zur Verbesserung der Immunfunktion nach einem der Ansprüche 1 bis 7.

10. Futtermittelprodukt, umfassend die Zusammensetzung zur Verbesserung der Immunfunktion nach einem der Ansprüche 1 bis 7.

11. Kosmetisches Produkt, umfassend die Zusammensetzung zur Verbesserung der Immunfunktion nach einem der Ansprüche 1 bis 7.

## Revendications

1. Composition pour améliorer la fonction immunitaire comprenant, comme ingrédients actifs, du zinc et un saccharide ayant le 3-O-α-D-glucopyranosyl-D-glucose en tant que motif structural, dans laquelle le saccharide est un nigérooligosaccharide comprenant au moins un parmi le nigérose, le nigérosyl glucose et le nigérosyl maltose.

2. Composition pour améliorer la fonction immunitaire selon la revendication 1, dans laquelle un rapport en poids du zinc au saccharide est de 1 :200 à 1 :2000.

3. Composition pour améliorer la fonction immunitaire selon la revendication 1 ou 2, dans laquelle l'amélioration de la fonction immunitaire est l'amélioration de l'activité métabolique d'une cellule immunocompétente.

4. Composition pour améliorer la fonction immunitaire selon la revendication 1 ou 2, dans laquelle l'amélioration de la fonction immunitaire est l'amélioration du potentiel de croissance d'une cellule immunocompétente.

5. Composition pour améliorer la fonction immunitaire selon la revendication 1 ou 2, dans laquelle l'amélioration de la fonction immunitaire est l'amélioration de l'activité métabolique d'un lymphocyte T.

6. Composition pour améliorer la fonction immunitaire selon la revendication 1 ou 2, dans laquelle l'amélioration de la fonction immunitaire est l'amélioration du potentiel de croissance d'un lymphocyte T.

7. Composition pour améliorer la fonction immunitaire selon la revendication 1 ou 2, dans laquelle l'amélioration de la fonction immunitaire est l'inhibition de la mort cellulaire d'un lymphocyte T.

8. Composition pour améliorer la fonction immunitaire selon l'une quelconque des revendications 1 à 7, qui est un produit pharmaceutique ou un produit alimentaire.

9. Produit alimentaire comprenant la composition pour améliorer la fonction immunitaire selon l'une quelconque des revendications 1 à 7.

10. Produit fourrager comprenant la composition pour améliorer la fonction immunitaire selon l'une quelconque des revendications 1 à 7.

11. Produit cosmétique comprenant la composition pour améliorer la fonction immunitaire selon l'une quelconque des revendications 1 à 7.
